# EUROPEAN PATENT SPECIFICATION

(11) **EP 4 487 778 B1**
(45) Date of publication and mention of the grant of the patent: **17.12.2025**
(21) Application number: 23182999.5
(22) Date of filing: 03.07.2023
(51) Int. Cl.: A61B 5/30, A61B 5/388, A61B 5/00

(54) **A CIRCUITRY AND A METHOD FOR RECORDING A BIOLOGICAL ELECTRICAL SIGNAL**
SCHALTUNG UND VERFAHREN ZUR AUFZEICHNUNG EINES BIOLOGISCHEN ELEKTRISCHEN SIGNALS
CIRCUIT ET PROCÉDÉ D'ENREGISTREMENT D'UN SIGNAL ÉLECTRIQUE BIOLOGIQUE

(43) Date of publication of application: 08.01.2025
(73) Proprietor: Stichting IMEC Nederland, 5656 AE Eindhoven (NL)
(72) Inventor: PETKOS, Konstantinos, 5615 AE Eindhoven (NL)
(74) Representative: AWA Sweden AB

(56) References cited:
- WO-A1-2004/000115
- US-A1- 2023 200 738
- US-B2- 10 206 596

## Description

### Technical field

The present description relates to a circuitry and method for recording a biological electrical signal.

### Background

It is widely accepted by the scientific community that electrical stimulation of specific target brain regions can alleviate symptoms of neurological disorders, such as Parkinson's disease, essential tremor, dystonia, epilepsy, Alzheimer's disease, depression, obsessive-compulsive disorder, Tourette's syndrome, etc. In recent years, the traditional practice of continuously stimulating the brain using static stimulation parameters has shifted to the use of disease biomarkers to determine intensity and timing of stimulation to be used. Biomarkers may thus be used for controlling stimulation such that stimulation may be provided only when required within a certain period of time. This may allow side effects of the stimulation to be reduced or minimized.

It would thus be desired to have a system allowing continuous monitoring of biological electrical signals such that disease biomarkers may be continuously identified. Hence, the concept of concurrent recording and stimulation of biological electrical signals is becoming more and more important in bidirectional stimulation and recording applications.

However, stimulation pulses create interference with readout circuits for recording biological electrical signals. The stimulation pulses may appear as artefacts masking the underlying biological electrical signal (such as a neural signal) and making concurrent recording and stimulation of biological electrical signals a challenge.

The artefacts may often be misclassified as action potentials by detection algorithms, and subsequent action potentials cannot be recorded until an amplifier for amplifying a sensed biological electrical signal has recovered from saturation. Thus, rejection or at least reduction of artefacts due to stimulation may be highly beneficial for providing improved accurate recordings of the bioelectrical signals of interest.

Patent publication WO 2004/000115 A1 shows prior art circuitry for recording biological electrical signals.

### Summary of the invention

It is an objective to, at least partly, mitigate, alleviate, or eliminate one or more of the above-identified deficiencies in the art singly or in any combination. In particular, it is an objective to provide recording of biological electrical signal while avoiding or reducing impact of stimulation signals on the recording of the biological electrical signals.

This and other objectives are at least partly met by the invention as defined in the independent claims. Preferred embodiments are set out in the dependent claims.

According to a first aspect, there is provided a circuitry for recording a biological electrical signal. The circuitry comprises a signal recording input configured to receive the biological electrical signal, a signal recording amplifier connected to the signal recording input and configured to provide an amplified biological electrical signal at an output node of the signal recording amplifier, a sampling circuit having an input node connected to the output node of the amplifier and configured to sample the amplified biological electrical signal, a feedback integrator connected to the input node of the sampling circuit and configured to provide a feedback signal into the signal recording amplifier, wherein the feedback integrator is configured to subtract a direct current (DC) offset from the amplified biological electrical signal. The circuitry further comprises a switch configured to selectively disconnect the output of the signal recording amplifier from the input node of the sampling circuit and a stimulation artefact detection block configured to detect a stimulation artefact, wherein the switch is configured to receive a control signal for controlling the switch, wherein the control signal is dependent on detection, by the stimulation artefact detection block, of a stimulation artefact affecting recording of the biological electrical signal.

Within the context of this disclosure the wording "biological electrical signal" should be construed as any electrical signal that can be acquired by any medical sensor or device configured to acquire electrical signals stemming from a biological source. The biological electrical signal may be an electrical signal acquired by an electrode or another sensor sensitive to electrical signals, wherein the electrode (or other type of sensor) is configured to detect the biological electrical signal propagating in a nerve, such as a nerve of the central nervous system or the peripheral nervous system. The biological electrical signal may be a signal constituting or forming part of electrocardiography, electroencephalography, electromyography, etc.

The circuitry of the first aspect allows for the circuit to disconnect the signal recording amplifier from the sampling circuit, whenever an artefact is detected by the stimulation artefact detection block. Put differently, the sampling circuit is only connected to the signal recording amplifier whenever the biological electrical signal does not exhibit an artefact, such that no artefacts or at least no significant artefacts are acquired by the sampling circuit.

The stimulation artefact detection block is configured to detect a stimulation artefact. Thus, the stimulation artefact detection block is able to identify timing of artefacts being present in the biological electrical signal received at the signal recording input. The switch is controlled such that, when the stimulation artefact detection block detects a stimulation artefact, the switch may disconnect the signal recording amplifier from the sampling circuit.

The stimulation artefact detection block may be configured to detect artefacts having a sufficient amplitude such that recording of the biological electrical signal is affected. Thus, the stimulation artefact detection block may for instance be configured to detect stimulation artefacts based on a threshold signal level being exceeded. It should be realized that even though the stimulation artefact detection block may be specifically used for controlling the switch in dependence of stimulation artefacts, the stimulation artefact detection block is not necessarily limited to a particular cause of an artefact that may affect recording of the biological electrical signal. Thus, if other artefacts cause increase in signal levels, such artefacts may also be detected by the signal artefact detection block and cause control of the switch.

The switch may be used for disconnecting the signal recording amplifier from the sampling circuit whenever a stimulation artefact due to simultaneous stimulation is detected. This enables preventing any stimulation artefacts from contaminating the signal of interest. More specifically, between stimulation pulses, the switch may normally connect the output node of the signal recording amplifier to the sampling circuit. Thus, the switch enables recording the biological electrical signal of interest through the sampling circuit. However, before the stimulation pulse affects the recording of biological electrical signal, the switch disconnects the output node of the signal recording amplifier from the sampling circuit, preventing the artefact from entering the sampling circuit and a signal chain for processing the biological electrical signal sampled by the sampling circuit. At end of a stimulation pulse, the switch reconnects the output node of the signal recording amplifier to the sampling circuit allowing recording of the biological electrical signal to continue until a next stimulation pulse arrives. In this way, any loss of biological electrical signal between the stimulation pulses is limited and the recorded signals are of high quality.

The circuitry also allows for reduction of DC offset of the biological electrical signal. This implies that a high quality recording of the actual biological electrical signal occurring in tissue. Acquisition of the biological electrical signal usually includes a strong DC offset stemming from the interface between the electrode and tissue. The feedback integrator connected to the input node of the sampling circuit may continuously extract the DC voltage level of the biological electrical signal received at the signal recording input and enables this offset to be subtracted from the received biological electrical signal in real time. As a result, a signal at the output node of the signal recording amplifier may be free from undesired DC offsets and may further be centered around a zero (common mode) voltage level.

The stimulation artefact detection block may further comprise a stimulation artefact detection input configured to receive a reference biological electrical signal corresponding to the biological electrical signal to be recorded and a stimulation artefact detection amplifier connected to the stimulation artefact detection input and configured to provide an amplified reference biological electrical signal at an output node of the stimulation artefact detection amplifier. The stimulation artefact block may further comprise a comparator circuit having an input node connected to the output node of the stimulation artefact detection amplifier and configured to compare the amplified reference biological electrical signal to a threshold, wherein the comparator circuit is arranged to detect the stimulation artefact based on the comparison of the amplified reference biological electrical signal and the specified threshold.

Thus, the stimulation artefact detection block may comprise a comparator circuit that compares the reference biological electrical signal to a specified threshold. The specified threshold may be set such that as soon as a signal level of the reference biological electrical signal is so large that recording of the biological electrical signal will be affected, the comparator circuit may provide a detection of the stimulation artefact. Thus, if the reference biological electrical signal exceeds the specified threshold the stimulation artefact detection block may determine that a stimulation artefact is present such that the switch may be appropriately controlled.

The use of the specified threshold may allow a simple comparison to be made by the comparator circuit. However, it should be realized that the stimulation artefact detection block may be implemented in other ways, for instance so as to analyze changes within the reference biological electrical signal.

It should be realized that the reference biological electrical signal may be related in different manners to the biological electrical signal received by the signal recording input such that the reference biological electrical signal corresponds to the biological electrical signal to be recorded. For instance, the reference biological electrical signal and the biological electrical signal to be recorded may be provided by the same sensor, such as the same electrode. Thus, the electrode may be connected both to the signal recording input and the stimulation artefact detection input, such that the reference biological electrical signal is the same as the biological electrical signal to be recorded. However, according to an alternative, different sensors may be used, which may be arranged at locations close to each other, such that the biological electrical signal acquired by a first sensor connected to the signal recording input may be very similar to the reference biological electrical signal acquired by a second sensor connected to the stimulation artefact detection input.

Furthermore, the stimulation artefact detection amplifier may comprise a feedback integrator connected to the input node of the comparator and configured to provide a feedback signal into the stimulation artefact detection amplifier, wherein the feedback integrator is configured to subtract a DC offset from the amplified reference biological electrical signal.

This implies that DC offsets may be removed or at least reduced from the reference biological electrical signal.

The comparator circuit may be connected to the switch and the switch may be configured to receive the control signal from the comparator circuit, wherein the control signal is based on the detection of the stimulation artefact by the comparator circuit.

Thus, the control signal for controlling the switch may be directly provided from the comparator circuit. This allows the control signal to be provided in a simple manner.

The control signal may control that the switch disconnects the output of the signal recording amplifier from the input node of the sampling circuit. Thus, the control signal provided by the comparator circuit may ensure that no artefacts enter the sampling circuit allowing the biological electrical signal to be recorded with high quality.

The circuitry may further comprise a current-limiting safety circuit block between the signal recording input and the signal recording amplifier.

This implies that safety of a living being from which the biological electrical signal is acquired increases for the circuitry. In particular, it should be realized that the current-limiting safety circuit block may be used with use of an electrode for detecting the biological electrical signal, wherein the electrode is connected to the signal recording input.

The current-limiting safety circuit block may comprise a resistor. The resistor may limit maximum DC and alternating current (AC) currents that can flow through the living being.

The current-limiting safety circuit block may further comprise a capacitor in parallel to the resistor. The capacitor may decrease an impact of the resistor on a recording performance at high frequencies.

The sampling circuit may comprise a low-pass filter configured to sample and hold the amplified biological electrical signal at the input node.

An associated advantage of this configuration is that the sampling circuit may ensure that there is no signal loss at the input node of the sampling circuit when the switch disconnects the output of the signal recording amplifier from the input node of the sampling circuit. When the switch disconnects the output of the signal recording amplifier from the input node of the sampling circuit, the sampling circuit may continue the hold the last recorded signal before interruption by the switch, until the switch is reconnected again.

The feedback integrator may comprise an operational amplifier and a feedback capacitor connected between an output of the operational amplifier and an inverting input of the operational amplifier for providing negative feedback to the operational amplifier.

An associated advantage of this configuration is that an operational amplifier may have a low power consumption, such that the circuitry may provide subtraction of DC offset in a power efficient manner. The operational amplifier may also be implemented in a compact manner such that a small space or footprint may be needed for incorporation of the operational amplifier in the circuitry.

The signal recording amplifier may be an instrumentation amplifier.

An associated advantage of this configuration is that an instrumentation amplifier may have an efficient suppression of common mode noise signals, as the instrumentation amplifier offers a high common mode rejection ratio.

An additional associated advantage of this configuration is that an instrumentation amplifier may have a low power consumption, such that the circuitry may provide subtraction of DC offset in a power efficient manner. The instrumentation amplifier may also be implemented in a compact manner such that a small space or footprint may be needed for incorporation of the instrumentation amplifier in the circuitry.

It should however be realized that the signal recording amplifier may alternatively be implemented as a differential amplifier.

The signal recording input may be a first signal recording input configured to receive a first biological electrical signal and wherein the circuitry further comprises a second signal recording input configured to receive a second biological electrical signal, wherein the signal recording amplifier may be a differential amplifier having an inverting input and a non-inverting input connected to the first signal recording input and the second signal recording input, respectively, wherein the feedback integrator may be connected to a reference input port of the signal recording amplifier.

An associated advantage of this configuration is that it provides differential signal recording of the biological electrical signal. This may be used for good attenuation of 50 Hz interference from mains supply.

The signal recording input may be a first signal recording input configured to receive a first biological electrical signal and wherein the first signal recording input may be connected to an inverting input of the signal recording amplifier, wherein the circuitry further comprises a second signal recording input configured to receive a second biological electrical signal, and wherein the circuitry further comprises a multiplexer, wherein the multiplexer is connected to the feedback integrator, and the multiplexer may be selectively controlled between a first and a second configuration.

In the first configuration, the feedback integrator may be connected via the multiplexer to a non-inverting input of the signal recording amplifier, and a reference input port of the signal recording amplifier is connected to a common-mode voltage. In the second configuration, the feedback integrator may be connected via the multiplexer to the reference input port of the signal recording amplifier, and the second signal recording input is connected to the non-inverting input of the signal recording amplifier.

An associated advantage of this configuration is that it provides a tunable and versatile circuitry with a first and a second configuration. Thus, it may be possible to tune the circuitry in line with different applications and intended uses of the circuitry.

In particular, the circuitry may be used in the first configuration to allow single-ended recording of the biological electrical signal.

In addition, the circuitry may be used in the second configuration to allow differential signal recording of the biological electrical signal. The feedback integrator may still be used for providing the DC offset to be subtracted from the amplified biological electrical signal by providing output from the feedback integrator at the reference input port of the signal recording amplifier.

The signal recording amplifier may be a first signal recording amplifier, the feedback integrator may be a first feedback integrator, the switch may be a first switch, the multiplexer may be a first multiplexer and the sampling circuit may be a first sampling circuit, wherein the first signal recording amplifier, the first feedback integrator, the first switch, the first multiplexer, the first sampling circuit and a second switch connected to an output of the first sampling circuit may form a first signal recording block, wherein the circuitry further comprises a second signal recording block comprising a second signal recording amplifier, a second feedback integrator, a second multiplexer, a second sampling circuit, a third switch configured to selectively disconnect the output of the second signal recording amplifier from an input node of the second sampling circuit, and a fourth switch connected to an output of the second sampling circuit, wherein the first and second switches may be configured to be de-activated in dependence of the detection, by the stimulation artefact detection block, of a signal level of the stimulation artefact being above a first threshold and the third and fourth switches are configured to be activated in dependence of the detection, by the stimulation artefact detection block, of the signal level of the stimulation artefact being above the first threshold.

An associated advantage of this configuration is that it allows signal loss during a monophasic stimulation to be avoided or reduced, as the configuration allows for continued acquirement of the biological electrical signal, even during a monophasic stimulation.

The second signal recording block may be activated to provide recording of the biological electrical signal during presence of the stimulation artefact.

The stimulation artefact detection block may further comprise a delay circuit for delaying a second control signal controlling the second switch in relation to a first control signal controlling the first switch and delaying a fourth control signal controlling the fourth switch in relation to a third control signal controlling the third switch.

An associated advantage of this configuration is that the delay ensures that feedback AC-coupled loops provided by the feedback integrators are allowed some time in order to properly settle before a signal is output from a respective sampling circuit. This may prevent any switching artefact from entering a signal chain for processing the biological electrical signals.

The circuitry may further comprise a third signal recording block comprising a third signal recording amplifier, a third feedback integrator, a third multiplexer, a third sampling circuit, a fifth switch configured to selectively disconnect the output of the third signal recording amplifier from an input node of the third sampling circuit, and a sixth switch connected to an output of the third sampling circuit, wherein the fifth and sixth switches may be configured to be activated in dependence of the detection, by the stimulation artefact detection block, of the signal level of the stimulation artefact being below a second threshold, lower than the first threshold.

An associated advantage of this configuration is it allows signal loss during a biphasic stimulation to be avoided or reduced, as the configuration allows for continued acquirement of the biological electrical signal, even during a stimulation pulse with two phases.

The second signal recording block may be activated to provide recording of the biological electrical signal during presence of a first phase of the stimulation artefact. The third signal recording block may be activated to provide recording of the biological electrical signal during presence of a second phase of the stimulation artefact.

According to a second aspect, there is provided an apparatus for recording a biological electrical signal, said apparatus comprising one or more electrodes for sensing the biological electrical signal and the circuitry according to the first aspect.

Effects and features of this second aspect are largely analogous to those described above in connection with the first aspect. Embodiments mentioned in relation to the second aspect are largely compatible with the first aspects.

Thus, the circuitry may be incorporated in an apparatus for recording a biological electrical signal. The apparatus may comprise electrodes for sensing the biological electrical signal and the circuitry for recording the biological electrical signal while avoiding or reducing impact of a stimulation artefact on the recording of the biological electrical signal.

The apparatus may further comprise a stimulation unit for providing a stimulation signal in a biological structure, e.g., in a nerve, such as a nerve in the central nervous system or the peripheral nervous system, or in a body region, such as a brain region, for stimulating activity of the biological electrical signal. Thanks to the circuitry being configured to avoid or reduce impact of stimulation artefacts, the apparatus is thus particularly suited for providing stimulation and simultaneously recording biological electrical signals.

According to a third aspect, there is provided a method for recording a biological electrical signal. The method comprises the steps of receiving, by a signal recording input, the biological electrical signal, amplifying, by a signal recording amplifier connected to the signal recording input, the biological electrical signal, sampling, by a sampling circuit having an input node connected to an output of the signal recording amplifier, the amplified biological electrical signal, providing, a feedback signal into the signal recording amplifier, wherein the feedback signal is created by subtracting a direct current (DC) offset, from the amplified biological electrical signal, using a feedback integrator connected to the input node of the sampling circuit, receiving, a control signal which is dependent of the detection of a stimulation artefact affecting recording of the biological electrical signal, in response to receiving the control signal, selectively disconnecting the output of the signal recording amplifier from the input node of the sampling circuit using a switch and recording, the biological electrical signal, when the switch connects the output of the signal recording amplifier to the input node of the sampling circuit for output of the amplified biological signal with a subtracted DC offset.

Effects and features of this third aspect are largely analogous to those described above in connection with the first and second aspects. Embodiments mentioned in relation to the third aspect are largely compatible with the first and second aspects.

The method allows recording the biological electrical signal while avoiding or reducing impact of a stimulation artefact on the recording of the biological electrical signal.

The method may further comprise the steps of generating the control signal, wherein said generating comprises receiving, by a stimulation artefact detection input, a reference biological signal corresponding to the biological signal to be recorded, amplifying, by a stimulation artefact detection amplifier connected to the stimulation artefact detection input, the reference biological electrical signal, comparing the amplified reference biological electrical signal to a specified threshold, generating the control signal in dependence on the comparison of the amplified biological electrical signal and the specified threshold.

Thus, the control signal for controlling the switch may be directly provided from the comparator circuit. This allows the control signal to be provided in a simple manner.

The method may further comprise switching, using one or more multiplexers, between two or more different circuit configurations for single-ended or differential recording of the biological electrical signal.

This allows tunable and versatile use of the method for recording the biological electrical signal.

### Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.
Fig. 1 is a schematic view of a circuitry for recording a biological electrical signal according to a first embodiment.
Fig. 2 is a schematic view a circuitry for recording a biological electrical signal according to a second embodiment.
Fig. 3 is a schematic view of a circuitry for recording a biological electrical signal according to a third embodiment.
Fig. 4 is a schematic view of a circuitry for recording a biological electrical signal according to a fourth embodiment.
Fig. 5 is a schematic view of a circuitry for recording a biological electrical signal according to a fifth embodiment.
Fig. 6 is a schematic view of an apparatus for recording a biological electrical signal.
Fig. 7 is a flow chart of a method for recording a biological electrical signal.

### Detailed description

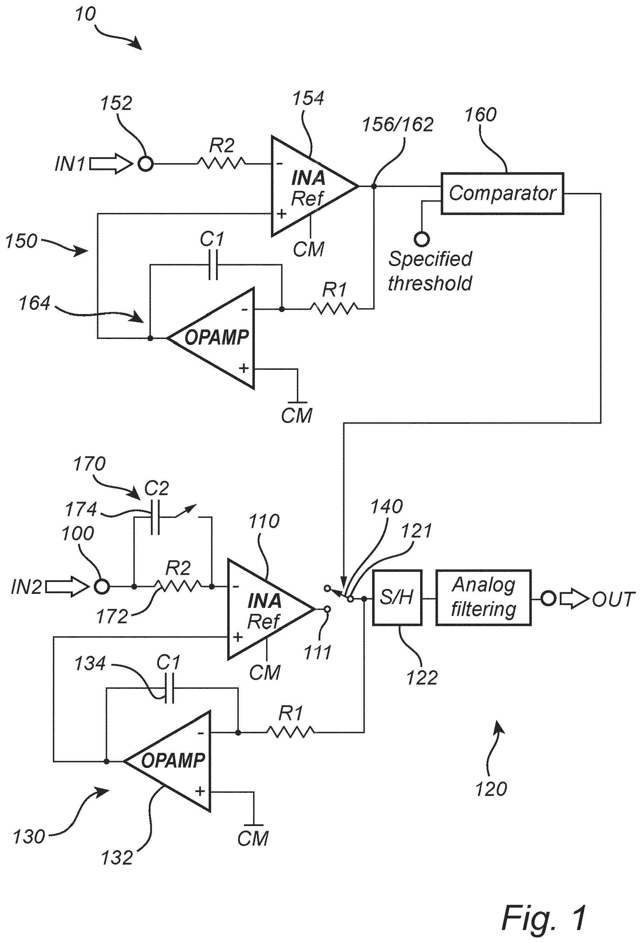

In the following, a circuitry 10 for recording a biological electrical signal according to several different embodiments will be described in connection with Figures 1-6. The circuitry 10 may form part of a medical device, e.g. in the form of a biomedical sensor. As illustrated in Fig. 1, the circuitry comprises a signal recording input 100, a signal recording amplifier 110, a sampling circuit 120, a feedback integrator 130, a switch 140, and a stimulation artefact detection block 150.

Referring now to Fig. 1, the circuitry 10 comprises a signal recording input 100 configured to receive a biological electrical signal. The biological electrical signal may be construed as any electrical signal that can be acquired by any medical sensor or device configured to acquire electrical signals originating from a biological source. Furthermore, the biological electrical signal may be an electrical signal acquired by an electrode or another sensor sensitive to electrical signals, wherein the electrode (or other type of sensor) is configured to detect the biological electrical signal propagating in a nerve, such as a nerve of the central nervous system or the peripheral nervous system. The biological electrical signal may be a signal constituting or forming part of electrocardiography, electroencephalography, electromyography, etc. In particular, the biological electrical signal may be an electrical signal propagating in a biological structure, such as in a nerve, in response to a stimulation of the biological structure.

The signal recording input 100 may be a node connected to the electrode or other sensor that is configured to acquire the biological electrical signal. The signal recording input 100 may thus form an input port connected to the electrode.

The circuitry 10 may further comprise a current-limiting safety circuit block 170 connected to the signal recording input 100. The current-limiting safety circuit block 170 may be arranged between the signal recording input 100 and the signal recording amplifier 110. The current-limiting safety circuit block 170 may provide safety of a living being from which the biological electrical signal is acquired. In particular, it should be realized that the current-limiting safety circuit block 170 may be used with use of an electrode for detecting the biological electrical signal S1, wherein the electrode is connected to the signal recording input 100. The current-limiting safety circuit block 170 may comprise a resistor 172. The resistor may limit maximum DC and AC currents that can flow through the living being. The current-limiting safety circuit block 170 may further comprise a capacitor 174 in parallel to the resistor 172. The capacitor 174 may decrease an impact of the resistor 172 on a recording performance at high frequencies. The capacitor 174 may mainly be useful when a bioimpedance signal is to be recorded and may therefore be associated with a switch for disconnecting the capacitor 174, which may allow the current-limiting safety circuit block 170 to be adapted to a type of biological electrical signal being detected.

A signal recording amplifier 110 is connected to the signal recording input 100 and configured to provide an amplified biological electrical signal at an output node 111 of the signal recording amplifier 110.

The signal recording amplifier 110 amplifies the biological electrical signal received at the signal recording input 100 into an amplified biological electrical signal. The amplified biological electrical signal may have an increased signal strength compared to the biological electrical signal received at the signal recording input 100, which may facilitate accurate measuring of the biological electrical signal. The signal recording amplifier 110 may be an instrumentation amplifier.

An associated advantage of this configuration may be that an instrumentation amplifier may have an efficient suppression of common mode noise signals, as the instrumentation amplifier offers a high common mode rejection ratio.

An additional associated advantage of this configuration is that an instrumentation amplifier may have a low power consumption, such that the circuitry 10 may provide subtraction of DC offset in a power efficient manner. The instrumentation amplifier may also be implemented in a compact manner such that a small space or footprint may be needed for incorporation of the instrumentation amplifier in the circuitry.

The circuitry 10 further comprises the feedback integrator 130, wherein the feedback integrator 130 is configured to provide a feedback signal into the signal recording amplifier 110. The feedback integrator 130 is configured to provide a feedback signal into the signal recording amplifier 110 so as to subtract a DC offset, whereby the DC offset is subtracted or at least reduced in the amplified biological electrical signal. The feedback integrator 130 continuously extracts the DC voltage level, e.g. the offset of the input AC signal of the amplified biological electrical signal using the feedback integrator 130. The feedback integrator feeds the extracted offset back into the signal recording amplifier 110 so as to subtract the offset from the amplified biological electrical signal in real time.

The feedback integrator 130 may be an analog feedback integrator. The feedback integrator 130 may comprise an operational amplifier 132 having an inverting input connected to receive the amplified biological electrical signal and a non-inverting input connected to a common-mode voltage. The feedback integrator 130 further comprises a feedback capacitor 134 connected between an output of the operational amplifier 132 and an inverting input of the operational amplifier for providing negative feedback to the operational amplifier 132.

An associated advantage of this configuration is that an operational amplifier may have a low power consumption, such that the circuitry 10 may provide subtraction of DC offset in a power efficient manner. The operational amplifier 132 may also be implemented in a compact manner such that a small space or footprint may be needed for incorporation of the operational 132 amplifier in the circuitry 10.

The feedback integrator 130 may comprise a resistor at the non-inverting input such that the amplified biological electrical signal passes the resistor before reaching the inverting input. The resistor may be tunable so that various high pass cut-off frequencies can be offered.

The feedback integrator 130 is configured to output a feedback signal into the signal recording amplifier 110. As shown in Fig. 1, the feedback signal may be provided at a non-inverting input of the signal recording amplifier 110. As a result of this, the output signal of the signal recording amplifier 110 is free from undesired DC offsets and properly centered around the zero (or common-mode) voltage level. Acquisition of the biological electrical signal usually includes a strong DC offset originating from the interface between the electrode and tissue. The feedback integrator 130 connected to receive the amplified biological electrical signal may continuously extract the DC voltage level of the biological electrical signal received at the signal recording input 100 and enables this offset to be subtracted from the received biological electrical signal in real time. As a result, a signal at the output node 111 of the signal recording amplifier 110 may be free from undesired DC offsets and may further be centered around a zero (common mode) voltage level.

The circuitry 10 further comprises the sampling circuit 120. The sampling circuit 120 has an input node 121 connected to the output node 111 of the signal recording amplifier 110. The sampling circuit 120 is configured to sample the amplified biological electrical signal.

The sampling circuit 120 may have a sample-and-hold (S/H) analog block 122 configured to receive the signal at the input node 121 of the sampling circuit 120. The S/H block 122 may prohibit signal loss if the incoming signal would be interrupted and reduces distortion levels of the recorded biological electrical signal.

The S/H block 122 may form a low-pass filter configured to sample and hold the amplified biological electrical signal at the input node 121. Thus the circuitry 10 allows for reduction of DC offset of the biological electrical signal. This implies that a high quality recording is provided of the actual biological electrical signal occurring in tissue.

The circuitry 10 further comprises the switch 140. The switch 140 is arranged between the output node 111 of the signal recording amplifier 110 and the input node 121 of the sampling circuit 120. The switch 140 is configured to selectively disconnect the output 111 of the signal recording amplifier 110 from the input node 121 of the sampling circuit 120. The switch 140 may be used for disconnecting the signal recording amplifier 110 from the sampling circuit 120 whenever a stimulation artefact is detected in the biological electrical signal. This enables preventing any stimulation artefacts from affecting detection of the signal of interest.

More specifically, the switch 140 may normally connect the output node 111 of the signal recording amplifier 110 to the input node 121 of the sampling circuit 120. Thus, the switch 140 enables recording of the biological electrical signal of interest through the sampling circuit 120. However, in case of a stimulation artefact being present in the biological electrical signal and affecting the recording of biological electrical signal, the switch 140 disconnects the output node 111 of the signal recording amplifier 110 from the sampling circuit 120. This prevents the artefact from entering the sampling circuit 120 and a signal chain for processing the biological electrical signal sampled by the sampling circuit 120. At end of a stimulation artefact, the switch 140 reconnects the output node 111 of the signal recording amplifier 110 to the input node 121 of the sampling circuit 120 allowing recording of the biological electrical signal to continue. In this way, any loss of biological electrical signal between stimulation artefacts, such as stimulation pulses, is limited and the recorded signals may be of high quality.

Thanks to the sampling circuit 120 having the S/H block 122 connected to the input node 121, the sampling circuit 120 may ensure that there is no signal loss when the switch 140 disconnects the output node 111 of the signal recording amplifier 110 from the input node 121 of the sampling circuit 120. When the switch 140 disconnects the output node 111 of the signal recording amplifier 110 from the input node 121 of the sampling circuit 120, the S/H block 122 may continue to hold the last recorded signal before interruption by the switch 140, until the switch 140 is reconnected again.

The feedback integrator 130 is connected to the input port 121 of the sampling circuit 120. When the switch 140 is closed, the feedback integrator 130 receives the amplified biological electrical signal for providing feedback to the signal recording amplifier 110. However, when the switch 140 is opened, the feedback integrator 130 is disconnected from the output node 111 of the signal recording amplifier 110.

The switch 140 is configured to receive a control signal, wherein the control signal S5 is dependent on a detection of a stimulation artefact. Thus, the switch 140 may be opened or closed in dependence of presence of a stimulation artefact in the biological electrical signal. The circuitry 10 further comprises the stimulation artefact detection block 150 which is configured to generate the control signal in dependence of detecting a stimulation artefact.

The stimulation artefact detection block 150 is configured to detect stimulation artefacts in the biological electrical signal. Thus, the stimulation artefact detection block 150 is able to identify timing of artefacts being present in the biological electrical signal received at the signal recording input 100. The switch 140 is controlled such that, when the stimulation artefact detection block 150 detects a stimulation artefact, the switch 140 may disconnect the signal recording amplifier 110 from the sampling circuit 120.

The stimulation artefact detection block 150 may be configured to detect artefacts having a sufficient amplitude such that recording of the biological electrical signal is affected. Thus, the stimulation artefact detection block 150 may for instance be configured to detect stimulation artefacts based on a threshold signal level being exceeded. It should be realized that even though the stimulation artefact detection block 150 may be specifically used for controlling the switch 140 in dependence of stimulation artefacts, the stimulation artefact detection block 150 is not necessarily limited to a particular cause of an artefact that may affect recording of the biological electrical signal. Thus, if other artefacts cause increase in signal levels, such artefacts may also be detected by the signal artefact detection block 150 and cause control of the switch 140. The circuitry 10 allows for disconnecting the signal recording amplifier 110 from the sampling circuit 120, whenever an artefact is detected by the stimulation artefact detection block 150. Put differently, the sampling circuit 120 is only connected to the signal recording amplifier 110 whenever the biological electrical signal does not exhibit an artefact, such that no artefacts or at least no significant artefacts are acquired by the sampling circuit 120.

The stimulation artefact detection block 150 may comprise a stimulation artefact detection input 152 configured to receive a reference biological electrical signal corresponding to the biological electrical signal to be recorded. The stimulation artefact detection block 150 further comprises a stimulation artefact detection amplifier 154 connected to the stimulation artefact detection input 152 and configured to provide an amplified reference biological electrical signal at an output node 156 of the stimulation artefact detection amplifier 154.

Furthermore, the stimulation artefact detection block 150 may comprise a feedback integrator 164, similar to the feedback integrator 130, implemented in connection to the signal recording amplifier 110. The feedback integrator 164 of the stimulation artefact detection block 150 is connected to the output node 156 of the stimulation artefact detection amplifier 154 and configured to provide a feedback signal into the stimulation artefact detection amplifier 154, wherein the feedback integrator is configured to subtract a DC offset from the amplified reference biological electrical signal. This implies that DC offsets may be removed or at least reduced from the reference biological electrical signal.

The stimulation artefact detection amplifier 154 may form a low-gain block having smaller gain than the signal recording amplifier110. This implies that amplifier saturation stemming from strong pulses due to stimulation artefacts may be avoided.

The stimulation artefact block 150 may further comprise a comparator circuit 160 having an input node 162 connected to the output node 156 of the stimulation artefact detection amplifier 154 and configured to compare the amplified reference biological electrical signal to a threshold, wherein the comparator circuit 160 is arranged to detect the stimulation artefact based on the comparison of the amplified reference biological electrical signal and the specified threshold.

Thus, the stimulation artefact detection block 150 may comprise a comparator circuit 160 that compares the reference biological electrical signal to a specified threshold. The specified threshold may be set such that as soon as a signal level of the reference biological electrical signal is so large that recording of the biological electrical signal will be affected, the comparator circuit 160 may provide a detection of the stimulation artefact. Thus, if the reference biological electrical signal exceeds the specified threshold the stimulation artefact detection block 150 may determine that a stimulation artefact is present such that the switch 140 may be appropriately controlled.

The use of the specified threshold may allow a simple comparison to be made by the comparator circuit 160. However, it should be realized that the stimulation artefact detection block 150 may be implemented in other ways, for instance so as to analyze changes within the reference biological electrical signal.

It should be realized that the reference biological electrical signal may be related in different manners to the biological electrical signal received by the signal recording input 100 such that the reference biological electrical signal corresponds to the biological electrical signal to be recorded. For instance, the reference biological electrical signal and the biological electrical signal to be recorded may be provided by the same sensor, such as the same electrode. Thus, the electrode may be connected both to the signal recording input 100 and the stimulation artefact detection input 152, such that the reference biological electrical signal is the same as the biological electrical signal to be recorded.

However, according to an alternative, different sensors may be used, which may be arranged at locations close to each other, such that the biological electrical signal acquired by a first sensor connected to the signal recording input 100 may be very similar to the reference biological electrical signal acquired by a second sensor connected to the stimulation artefact detection input 152.

The comparator circuit 160 may be connected to the switch 140 and the switch 140 may be configured to receive the control signal from the comparator circuit 160, wherein the control signal is based on the detection of the stimulation artefact by the comparator circuit 160.

Thus, the control signal for controlling the switch 140 may be directly provided from the comparator circuit 160. This allows the control signal to be provided in a simple manner.

The control signal may control that the switch 140 disconnects the output 111 of the signal recording amplifier 110 from the input node 121 of the sampling circuit 120. Thus, the control signal provided by the comparator circuit 160 may ensure that no artefacts enter the sampling circuit 120 allowing the biological electrical signal S1 to be recorded with high quality.

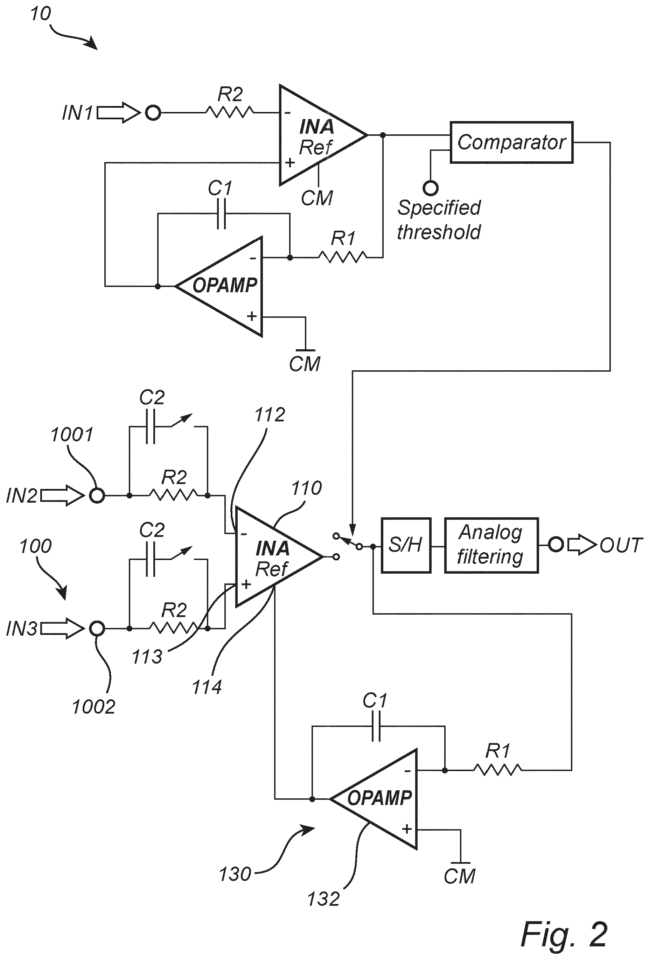

Referring now to Fig. 2, the circuitry 10 according to a second embodiment may comprise a plurality of signal recording inputs. As such, the signal recording input 100 may comprise a first signal recording input 1001 configured to receive a first biological electrical signal and wherein the circuitry 10 further comprises a second signal recording input 1002 configured to receive a second biological electrical signal. The signal recording amplifier 110 may be a differential amplifier having an inverting input 112 and a non-inverting input 113 connected to the first signal recording input 1001 and the second signal recording input 1002, respectively. Thus, the signal recording amplifier 110 may be configured to amplify a differential signal based on input from two sensors. Further, the feedback integrator 130 may be connected to a reference input port 114 of the signal recording amplifier 110 so as to provide the feedback signal into the reference input port 114 of the signal recording amplifier 110 whereby the DC offset may be subtracted from the amplified biological electrical signal.

The circuitry 10 shown in Fig. 2 may be identical to the circuitry shown in Fig. 1, except for the signal recording amplifier 110 receiving the second biological electrical signal instead at the non-inverting input 113 and receiving the feedback signal at the reference input port 114, instead of the feedback signal being received at the non-inverting input.

An associated advantage of this configuration is that it provides differential signal recording of biological electrical signals. This may be used for good attenuation of 50 Hz interference from mains supply.

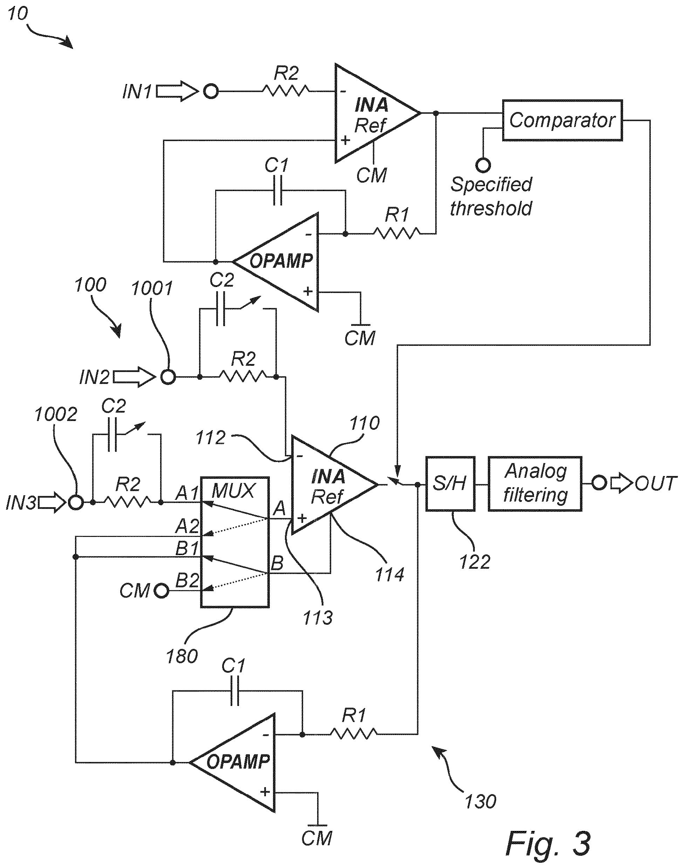

Referring now to Fig. 3, the circuitry 10 according to a third embodiment will be described. The circuitry 10 of the third embodiment corresponds to the circuitry of the second embodiment except for additional components being provided. The components that are the same as in the circuitry of the first and second embodiments will not be further described herein.

Like the circuitry of the second embodiment, the circuitry 10 of the third embodiment has a signal recording input 100 that comprises a first signal recording input 1001 configured to receive a first biological electrical signal and a second signal recording input 1002 configured to receive a second biological electrical signal. The first signal recording input 1001 is connected to an inverting input 112 of the signal recording amplifier 110.

However, in contrast to the circuitry of the second embodiment, the circuitry 10 of the third embodiment comprises a multiplexer 180. The second signal recording input 1002 is connected to a first input of the multiplexer 180. The feedback integrator 130 is also connected to output the feedback signal at a second input of the multiplexer 180. Further, a common-mode voltage is connected to a third input of the multiplexer 180. The multiplexer 180 further comprises a first output connected to the non-inverting input 113 of the signal recording amplifier 110 and a second output connected to the reference input port 114 of the signal recording amplifier 110.

The multiplexer 180 may be selectively controlled between a first and a second configuration.

In the first configuration, the feedback integrator 130 is connected via the multiplexer 180 to the non-inverting input 113 of the signal recording amplifier 110, and the reference input port 114 of the signal recording amplifier 110 is connected to the common-mode voltage. In the second configuration, the feedback integrator 130 may be connected via the multiplexer 180 to the reference input port 114 of the signal recording amplifier 110, and the second signal recording input 1002 is connected to the non-inverting input 113 of the signal recording amplifier 110.

An associated advantage of this configuration is that it provides a tunable and versatile circuitry with a first and a second configuration. Thus, it may be possible to tune the circuitry 10 in line with different applications and intended uses of the circuitry.

In particular, the circuitry 10 may be used in the first configuration to allow single-ended recording of the biological electrical signal.

In addition, the circuitry 10 may be used in the second configuration to allow differential signal recording of the biological electrical signal. The feedback integrator 130 may still be used for providing the DC offset to be subtracted from the amplified biological electrical signal by providing output from the feedback integrator 130 at the reference input port 114 of the signal recording amplifier 110.

More specifically, the circuitry 10 shown in Fig. 3 can be tuned or setup such that it can take the form of the circuitry presented in Figure 1. This may be achieved by connecting the second input of the multiplexer 180 to the first output and the third input of the multiplexer 180 to the second output. The circuitry 10 shown in Fig. 3 may alternatively be tuned to take the form of the circuitry presented in Fig. 2. This may be achieved by connecting the first input of the multiplexer 180 to the first output and the second input of the multiplexer 180 to the second output.

As shown in Fig. 3, resistors may be placed in the circuitry 10 at the first and second signal recording input, 1001 and 1002 respectively. This may provide current-limiting safety circuit blocks for both inputs 1001, 1022 in order to increase protection of the human or animal being from which biological electrical signals are acquired.

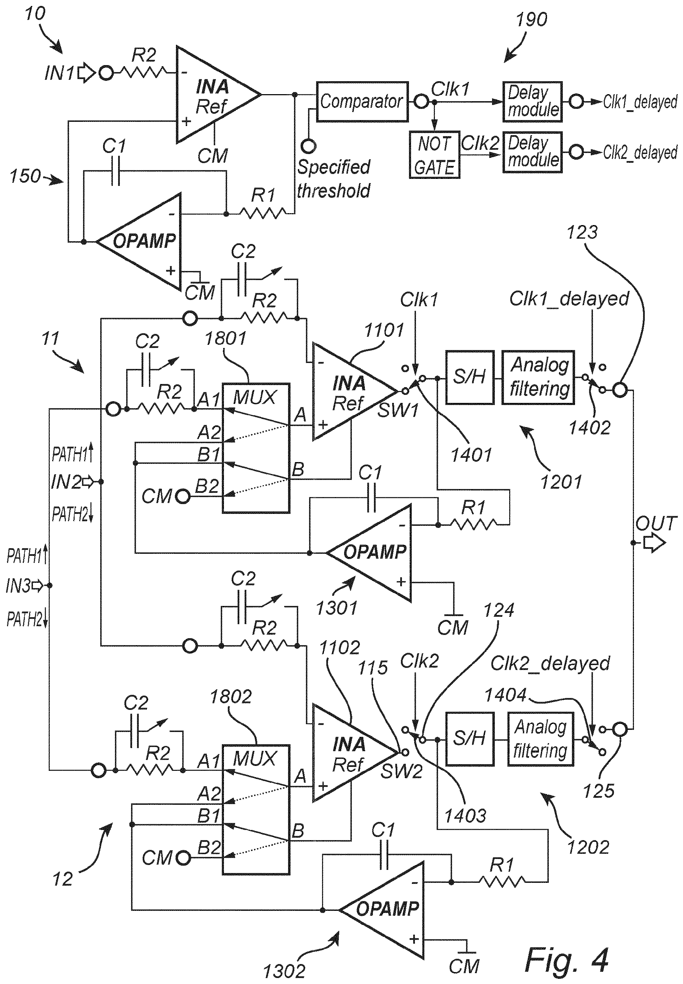

Referring now to Fig. 4, the circuitry 10 according to a fourth embodiment will be described. The circuitry 10 of the fourth embodiment corresponds to the circuitry of the third embodiment except for additional components being provided. The components that are the same as in the circuitry of the third embodiment will not be further described herein.

In the circuitry 10 of the fourth embodiment, the signal recording amplifier 100 as shown in the first, second and third embodiments is a first signal recording amplifier 1101, the feedback integrator 130 is a first feedback integrator 1301, the switch 140 is a first switch 1401, the multiplexer 180 is a first multiplexer 1801 and the sampling circuit 120 is a first sampling circuit 1201. The first signal recording amplifier 1101, the first feedback integrator 1301, the first switch 1401, the first multiplexer 1801, the first sampling circuit 1201 and a second switch 1402 connected to an output 123 of the first sampling circuit 1201 may form a first signal recording block 11.

The circuitry 10 further comprises a second signal recording block 12 comprising a second signal recording amplifier 1102, a second feedback integrator 1302, a second multiplexer 1802, a second sampling circuit 1202, a third switch 1403 configured to selectively disconnect the output 115 of the second signal recording amplifier 1102 from an input node 124 of the second sampling circuit 1202, and a fourth switch 1404 connected to an output 125 of the second sampling circuit 1202. The second signal recording block 12 may be identical to the first signal recording block 11. The first signal recording block 11 and the second signal recording block 12 may be selectively activated via the first, second, third and fourth switches 1401, 1402, 1403, 1404 such that the first signal recording block 11 may be active when no stimulation artefact is present, whereas the second signal recording block 12 may be active when a stimulation artefact is present.

Thus, the first and second switches, 1401 and 1402 respectively, may be configured to be de-activated in dependence of the detection, by the stimulation artefact detection block 150, of a signal level of the stimulation artefact S4 being above a first threshold, such that the output of the first signal recording amplifier 1101 is disconnected from the first sampling circuit 1201 and the output of the first sampling circuit 1201 is disconnected from providing an output from the circuitry 10. The third and fourth switches, 1403 and 1404 respectively, are configured to be activated in dependence of the detection, by the stimulation artefact detection block 150, of the signal level of the stimulation artefact being above the first threshold.

The de-activation of the first signal recording block 11 when a stimulation artefact arrives in the biological electrical signal ensures that the first signal recording block 11 will not get contaminated by a short, large DC offset associated with the stimulation artefact. The second signal recording block 12 is however inactive before the stimulation artefact arrives. Hence, the second signal recording block 12 only receives a large DC offset with a biological electrical signal superposed on the DC offset. Thus, the second signal recording block 12 is able to reject the DC offset and may output a biological electrical signal which is free from DC offset and stimulation artefact.

An associated advantage of this configuration is that it allows for signal loss during a monophasic stimulation to be avoided or reduced, as the configuration allows for continued acquirement of the biological electrical signal, even during a monophasic stimulation. The second signal recording block 12 may be activated to provide recording of the biological electrical signal during presence of the stimulation artefact.

The architecture of the circuitry 10, as illustrated in Fig. 4 may be used as a way to minimize any loss of the biological electrical signal during a monophasic stimulation. It may provide a way of continuing the recording of the biological electrical signal, even during the stimulus pulse interval.

This may be achieved by using the different switches of circuitry 10 to divert the incoming biological electrical signal along a desired path. The first switch 1401 and the second switch 1402 may be activated, allowing the biological electrical signal to pass through a first path in the first signal recording block 11 from input to output of the circuitry 10 when stimulation is not present. During this period, a second path through the second signal recording block 12, comprising the third switch 1403 and the fourth switch 1404, is disconnected from both the input and the output of the circuitry 10, closing the second path from any incoming signal.

When a stimulation artefact is present, the second signal path through the second signal recording block 12 gets activated by the third switch 1403 and the fourth switch 1404 being activated, while the first signal path through the first signal recording block 11 is disconnected from both the input and the output of the circuitry 10. Thus, during normal recording of the biological electrical signal, the signal is directed along the first path through the first signal recording block 11 and during the period of stimulation, the signal is directed along the second path through the second signal recording block 12.

The stimulation artefact detection block 150 may be configured to provide two different clock signals. The output from the comparator 160 may form a first clock signal. The output from the comparator 160 may further be connected to a NOT gate for inverting the output. The output from the NOT gate may form a second clock signal.

The first clock signal may control the first and second switches 1401 and 1402 and the second clock signal may control the third and fourth switches 1403 and 1404.

However, as shown in Fig. 4, the stimulation artefact detection block 150 may further comprise a delay circuit 190. The delay circuit 190 may delay the first clock signal to form a second control signal controlling the second switch 1402 which is slightly delayed in relation to the first clock signal that forms a first control signal controlling the first switch 1401. The delay circuit 190 may also delay the second clock signal to form a fourth control signal controlling the fourth switch 1404 which is slightly delayed in relation to the second clock signal that forms a third control signal controlling the third switch 1403.

When the stimulation pulse enters the signal path, the switch 1401 is deactivated and switch 1403 is activated. Switch 1402 may be deactivated slightly after the deactivation of switch 1401, and switch 1404 may be activated slightly after the activation of switch 1403. This strategy provides the feedback AC-coupled loops with some time in order to properly settle, and thus prevents switching artefacts from entering the signal chain.

Similarly, when the stimulation pulse duration ends switch 1403 is deactivated and switch 1401 is activated. Switch 1404 may be deactivated slightly after the deactivation of switch 1403, and switch 1402 may be activated slightly after the activation of switch 1401 to provide the feedback AC-coupled loops with some time in order to properly settle, and thus prevent the switching artefact from entering the signal chain.

Two different sample-and-hold blocks may be added in the two paths in order to maintain the voltage at a stable level. The voltage may be equal to the last recorded value. This may be important during the short intervals where switch 1403 and switch 1402 are both deactivated. This may occur during the period of stimulation termination and the switches are waiting for the AC-coupled feedback loops to properly settle. Similarly, this may occur during the short intervals where switch 1401 and switch 1404 are both deactivated, when the stimulation is about to be activated, and waiting for the AC-coupled feedback loops to properly settle. Analog low-pass filtering blocks may also be added to remove any remaining switching artefacts and high-frequency noise aggressors.

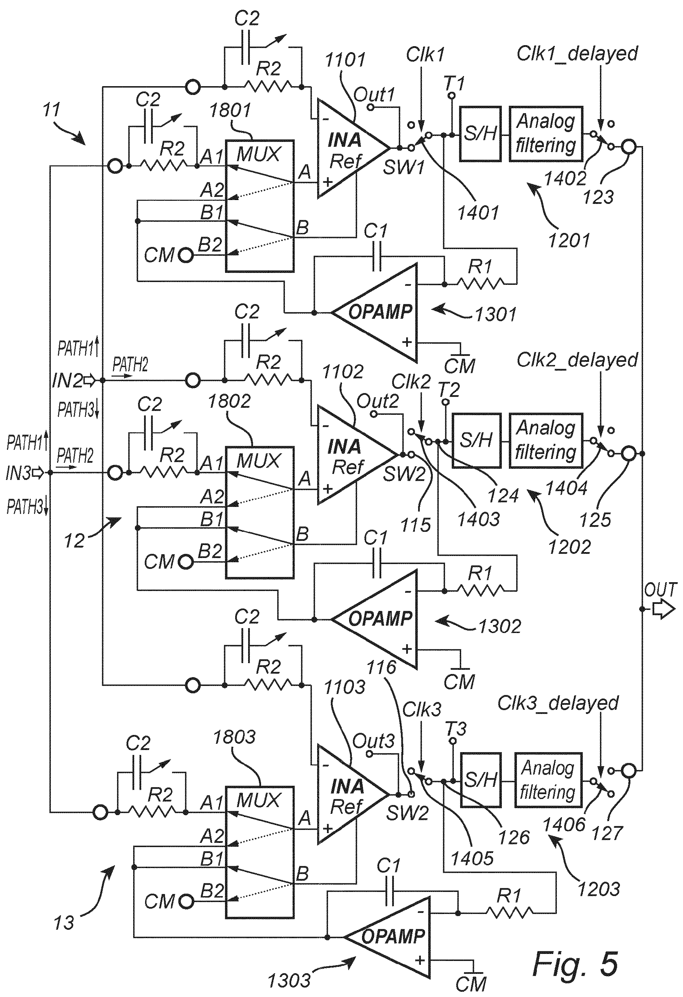

Referring now to Fig. 5, the circuitry 10 according to a fifth embodiment will be described. The circuitry 10 of the fifth embodiment corresponds to the circuitry of the fourth embodiment except for additional components being provided. The components that are the same as in the circuitry of the fourth embodiment will not be further described herein.

In addition to the first signal recording block 11 and the second signal recording block 12, the circuitry 10 may further comprise a third signal recording block 13 comprising a third signal recording amplifier 1103, a third feedback integrator 1303, a third multiplexer 1803, a third sampling circuit 1203, a fifth switch 1405 configured to selectively disconnect the output 116 of the third signal recording amplifier 1103 from an input node 126 of the third sampling circuit 1203, and a sixth switch 1406 connected to an output 127 of the third sampling circuit 1203, wherein the fifth and sixth switches, 1405 and 1406 respectively, may be configured to be activated in dependence of the detection, by the stimulation artefact detection block 150, of the signal level of the stimulation artefact S4 being below a second threshold, lower than the first threshold.

An associated advantage of this configuration is it allows signal loss during a biphasic stimulation to be avoided or reduced, as the configuration allows for continued acquirement of the biological electrical signal, even during a stimulation pulse with two phases. The second signal recording block 12 may be activated to provide recording of the biological electrical signal during presence of a first phase of the stimulation artefact. The third signal recording block 13 may be activated to provide recording of the biological electrical signal during presence of a second phase of the stimulation artefact.

The architecture of the circuitry 10, as illustrated in Fig 5. may be used as a way to minimize signal loss of the biological electrical signal during biphasic stimulation, since it provides a way of continuing the recording of the biological electrical signal, even during the two phases of the stimulus pulse. The two different phases may be anodic and cathodic phase.

As shown in Fig. 5, the circuitry 10 now comprises three parallel paths through the first signal recording block 11, through the second signal recording block 12, and through the third signal recording block 13, respectively, between an input and an output of the circuitry 10. The three signal recording blocks 11, 12, 13 may be identical.

The circuitry 10 may use the different switches to divert the incoming biological electrical signal along a desired path. The circuitry 10 may be configured to activate the fifth switch 1405 and the sixth switch 1406, allowing the signal to pass through when a cathodic phase of stimulation is present. During this period, the first and second path, comprising the signal paths of switches 1401 and 1402 in addition to switches 1403 and 1404, are disconnected from both the input and the output of the circuitry 10, closing the two signal paths from any incoming signal.

Prior to the initiation of the anodic phase of stimulation, either the first or the third signal path may be activated, while the switches of the remaining signal paths are disconnected from both the input and the output of the circuitry 10. During anodic phase of stimulation, the signal path of the third switch 1403 and the fourth switch 1404 is activated, while the first, second, fifth, and sixth switches 1401, 1402, 1405 and 1406 are disconnected.

Thus, as an example, during normal recording of the biological electrical signal, the biological electrical signal may be directed along the path of the first switch 1401 and the second switch 1402. During the period of anodic stimulation, the biological electrical signal may be directed along the path of the third switch 1403 and the fourth switch 1404 and during the period of cathodic stimulation, the biological electrical signal may be directed along the path of the fifth switch 1405 and the sixth switch 1406. An added benefit of the second, fourth and sixth switches 1402, 1404 and 1406 may be the removal of switching artefacts in a similar manner as described above in the fourth embodiment.

More specifically, when no stimulation signal is present, the first switch 1401 and the second switch 1402 may be activated whereas all the remaining switches are disconnected. However, when the stimulation starts with an anodic pulse, the first switch 1401 may be deactivated while the third switch 1403 is activated. In this case, the second switch 1402 can be deactivated slightly after the deactivation of the first switch 1401, and the fourth switch 1404 may be activated slightly after the activation of the third switch 1403 in an effort to provide the feedback AC-coupled loops with some time in order to properly settle, and thus prevent the switching artefact from entering the signal chain.

Following the stimulation with an anodic pulse, a cathodic pulse of stimulation may be provided. Thus, following the stage of anodic stimulation, the third switch 1403 is deactivated and the fifth switch 1405 is activated. In this case, the fourth switch 1404 may be deactivated slightly after the deactivation of the third switch 1403, and the sixth switch 1406 can be activated slightly after the activation of the fifth switch 1405 to provide the feedback AC-coupled loops with some time in order to properly settle, and thus prevent the switching artefact from entering the signal chain.

Similarly, when the cathodic stimulation pulse duration ends, the fifth switch 1405 is deactivated and the first switch 1401 is activated again. In this case, the sixth switch 1406 may be deactivated slightly after the deactivation of the fifth switch 1405, and the second switch 1402 may be activated slightly after the activation of the first switch 1401 to provide the feedback AC-coupled loops with some time in order to properly settle, and thus prevent the switching artefact from entering the signal chain.

The stimulation artefact detection block 150 may comprise a delay circuit similar to the delay circuit shown in Fig. 4 in order to generate the delayed signals to the second, fourth and sixth switches 1402, 1404, 1406.

Three different sample-and-Hold blocks may be added in the three paths in order to maintain the voltage at a stable level. The voltage may be equal to the last recorded value at a stable level. This may be important during the short interval where the first switch 1401 and the fourth switch 1404 are both deactivated, at the point of stimulation onset with an anodic pulse and waiting for the AC-coupled feedback loops to properly settle. This may also occur during the short interval where the third switch 1403 and the sixth switch 1406 are both deactivated, at the point of the switching from the anodic to the cathodic pulse phase and waiting for the AC-coupled feedback loops to properly settle. In a similar fashion this may also occur during the short period where the fifth switch 1405 and the second switch 1402 are both deactivated, at the point of biphasic pulse termination, and waiting for the AC-coupled feedback loops to properly settle.

Finally, analog low-pass filtering blocks can also be added here to remove any remaining switching artefacts and high-frequency noise aggressors. In addition, a first order high pass filter may be included in each path to remove any DC offsets that can appear and distort the recorded biological electrical signal.

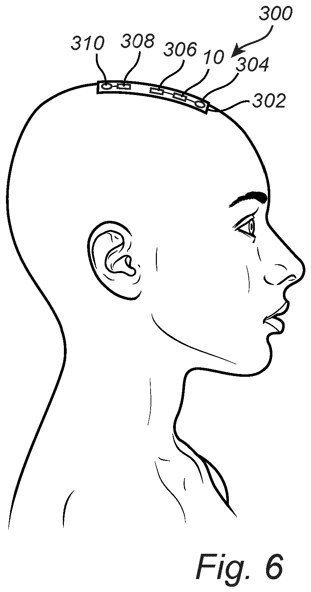

Referring now to Fig. 6, an apparatus 300 for recording a biological electrical signal will be described. The circuitry 10 according to any of the above-described embodiments may be incorporated in the apparatus 300.

The apparatus 300 may comprise a carrier 302 which may be configured to be arranged in relation to a biological structure in which a biological electrical signal is to be sensed. For instance, the carrier 302 may be adapted for being placed externally, e.g. in contact with the biological structure, such that the apparatus 300 may be a wearable device, or inserted partly or entirely into the biological structure, such that the apparatus 300 may be adapted to be implanted into a body.

The apparatus 300 may for instance be configured to sense the biological electrical signal propagating in a nerve, such as a nerve of the central nervous system or the peripheral nervous system. As illustrated in Fig. 6, the apparatus 300 may be configured to be arranged in relation to a brain for detecting biological electrical signals in the brain. Thus, the carrier 302 may for instance be a patch.

The apparatus 300 further comprises at least one electrode 304, which is configured to detect the biological electrical signal propagating in a nerve, such as a nerve of the central nervous system or the peripheral nervous system. The signals sensed by the at least one electrode 304 may be provided as input to the circuitry 10. The at least one electrode 304 may be arranged on a surface of the carrier 302 in order for the at least one electrode 304 to be arranged to detect the biological electrical signal.

The apparatus 300 may further comprise an analog-to-digital converter, which may be configured to convert the recorded biological electrical signals from the circuitry 10 to a digital form. The apparatus 300 may also comprise a processing unit 306, which may be configured to further process the recorded biological electrical signal. The processing unit 306 may also be arranged in the carrier 302. However, it should be realized that the apparatus 300 may be configured to transmit recorded biological electrical signals to an external unit for further processing therein.

The apparatus 300 may further comprise a stimulation unit 308 for providing a stimulation signal into the biological structure. Thus, the apparatus 300 may be configured to provide stimulation to the biological structure. The stimulation signal may be an electrical signal which may cause propagation of the biological electrical signal in the biological structure. The stimulation unit 308 may be connected to at least one stimulation electrode 310 for providing the stimulation signal into the biological structure. The stimulation electrode 310 may be arranged on the surface of the carrier 302.

Thanks to the circuitry 10 being able to avoid or reduce impact of a stimulation artefact on the recording of the biological electrical signal, the apparatus 300 is suited for simultaneous stimulation and recording of biological electrical signals.

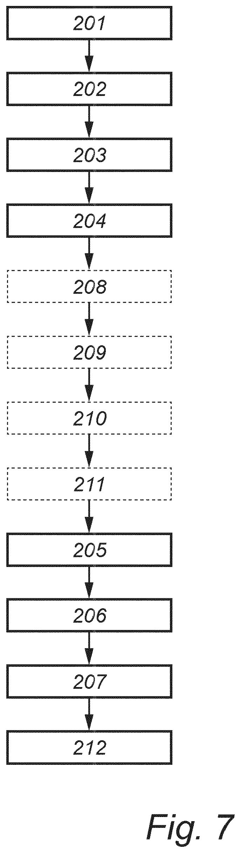

Referring now to Fig. 7, a method 20 for recording a biological electrical signal will be described. The method 20 may be implemented in a circuitry, e.g. the circuitry 10 as illustrated in any of Figs 1-5.

The method 20 comprises the steps of receiving 201, by a signal recording input, the biological electrical signal, amplifying 202, by a signal recording amplifier connected to the signal recording input, the biological electrical signal, sampling 203, by a sampling circuit having an input node connected to an output of the signal recording amplifier, the amplified biological electrical signal, providing 204, a feedback signal into the signal recording amplifier, wherein the feedback signal subtracts a DC offset, from the amplified biological electrical signal, using a feedback integrator connected to the input node of the sampling circuit, receiving 205, a control signal which is dependent of the detection of a stimulation artefact affecting recording of the biological electrical signal, in response to receiving the control signal, selectively disconnecting 206 the output of the signal recording amplifier from the input node of the sampling circuit using a switch and recording 207, the biological electrical signal, when the switch connects the output of the signal recording amplifier to the input node of the sampling circuit for output of the amplified biological signal S2 with a subtracted DC offset.

Effects and features of method are largely analogous to those described above in connection with the effects and features of circuitry 10. The method 20 allows recording the biological electrical signal while avoiding or reducing impact of a stimulation artefact on the recording of the biological electrical signal.

The method may further comprise the steps of generating the control signal, wherein said generating comprises receiving 208, by a stimulation artefact detection input, a reference biological signal corresponding to the biological signal to be recorded, amplifying 209, by a stimulation artefact detection amplifier connected to the stimulation artefact detection input, the reference biological electrical signal, comparing 210 the amplified reference biological electrical signal to a specified threshold, generating 211 the control signal in dependence on the comparison of the amplified biological electrical signal and the specified threshold. Thus, the control signal for controlling the switch may be directly provided from the comparator circuit. This allows the control signal to be provided in a simple manner.

The method may further comprise switching 212, using one or more multiplexers, between two or more different circuit configurations for single-ended or differential recording of the biological electrical signal. This allows tunable and versatile use of the method for recording the biological electrical signal.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A circuitry (10) for recording a biological electrical signal, said circuitry (10) comprising:
a signal recording input (100) configured to receive the biological electrical signal;
a signal recording amplifier (110) connected to the signal recording input (100) and configured to provide an amplified biological electrical signal at an output node (111) of the signal recording amplifier (110);
a sampling circuit (120) having an input node (121) connected to the output node (111) of the amplifier (110) and configured to sample the amplified biological electrical signal;
a switch (140) configured to selectively disconnect the output (111) of the signal recording amplifier (110) from the input node (121) of the sampling circuit (120); and
a stimulation artefact detection block (150) configured to detect a stimulation artefact;
wherein the switch (140) is configured to receive a control signal for controlling the switch (140), wherein the control signal is dependent on detection, by the stimulation artefact detection block (150), of a stimulation artefact affecting recording of the biological electrical signal,**characterized in that** the circuitry further comprises a feedback integrator (130) connected to the input node (121) of the sampling circuit (120) and configured to provide a feedback signal into the signal recording amplifier (110), wherein the feedback integrator (130) is configured to subtract a direct current, DC, offset from the amplified biological electrical signal.

2. The circuitry (10) according to claim 1, wherein the stimulation artefact detection block (150) comprises:
a stimulation artefact detection input (152) configured to receive a reference biological electrical signal corresponding to the biological electrical signal to be recorded;
a stimulation artefact detection amplifier (154) connected to the stimulation artefact detection input (152) and configured to provide an amplified reference biological electrical signal at an output node (156) of the stimulation artefact detection amplifier (154);
a comparator circuit (160) having an input node (162) connected to the output node (156) of the stimulation artefact detection amplifier (154) and configured to compare the amplified reference biological electrical signal to a threshold, wherein the comparator circuit (160) is arranged to detect the stimulation artefact based on the comparison of the amplified reference biological electrical signal and the specified threshold.

3. The circuitry (10) according to claim 2, wherein the comparator circuit (160) is connected to the switch (140); and
wherein the switch (140) is configured to receive the control signal from the comparator circuit (160), wherein the control signal is based on the detection of the stimulation artefact by the comparator circuit (160).

4. The circuitry (10) according to any one of the preceding claims, further comprising a current-limiting safety circuit block (170) between the signal recording input (100) and the signal recording amplifier (110).

5. The circuitry (10) according to any one of the preceding claims, wherein the sampling circuit (120) comprises a low-pass filter (122) configured to sample and hold the amplified biological electrical signal at the input node (121).

6. The circuitry (10) according to any one of the preceding claims, wherein the feedback integrator (130) comprises an operational amplifier (132) and a feedback capacitor (134) connected between an output of the operational amplifier and an inverting input of the operational amplifier for providing negative feedback to the operational amplifier (132).

7. The circuitry (10) according to any one of the preceding claims, wherein the signal recording input (100) is a first signal recording input (1001) configured to receive a first biological electrical signal and wherein the circuitry (10) further comprises a second signal recording input (1002) configured to receive a second biological electrical signal, wherein the signal recording amplifier (110) is a differential amplifier having an inverting input (112) and a non-inverting input (113) connected to the first signal recording input (1001) and the second signal recording input (1002), respectively, wherein the feedback integrator (130) is connected to a reference input port (114) of the signal recording amplifier (110).

8. The circuitry (10) according to any one of claims 1-6, wherein the signal recording input (100) is a first signal recording input (1001) configured to receive a first biological electrical signal and wherein the first signal recording input (1001) is connected to an inverting input (112) of the signal recording amplifier (110), wherein the circuitry (10) further comprises a second signal recording input (1002) configured to receive a second biological electrical signal, and wherein the circuitry (10) further comprises a multiplexer (180), wherein the multiplexer (180) is connected to the feedback integrator (130), and the multiplexer (180) is selectively controlled between a first and a second configuration;
wherein, in the first configuration, the feedback integrator (130) is connected via the multiplexer (180) to a non-inverting input (113) of the signal recording amplifier (110), and a reference input port (114) of the signal recording amplifier (110) is connected to a common-mode voltage, and
wherein, in the second configuration, the feedback integrator (130) is connected via the multiplexer (180) to the reference input port (114) of the signal recording amplifier (110), and the second signal recording input (1002) is connected to the non-inverting input (113) of the signal recording amplifier (110).

9. The circuitry (10) according to claim 8, wherein the signal recording amplifier (110) is a first signal recording amplifier (1101), the feedback integrator (130) is a first feedback integrator (1301), the switch (140) is a first switch (1401), the multiplexer (180) is a first multiplexer (1801) and the sampling circuit (120) is a first sampling circuit (1201), wherein the first signal recording amplifier (1101), the first feedback integrator (1301), the first switch (1401), the first multiplexer (1801), the first sampling circuit (1201) and a second switch (1402) connected to an output (123) of the first sampling circuit (1201) form a first signal recording block (11), wherein the circuitry (10) further comprises a second signal recording block (12) comprising a second signal recording amplifier (1102), a second feedback integrator (1302), a second multiplexer (1802), a second sampling circuit (1202), a third switch (1403) configured to selectively disconnect the output (115) of the second signal recording amplifier (1102) from an input node (124) of the second sampling circuit (1202), and a fourth switch (1404) connected to an output (125) of the second sampling circuit (1202), wherein the first and second switches (1401, 1402) are configured to be de-activated in dependence of the detection, by the stimulation artefact detection block (150), of a signal level of the stimulation artefact being above a first threshold and the third and fourth switches (1403, 1404) are configured to be activated in dependence of the detection, by the stimulation artefact detection block (150), of the signal level of the stimulation artefact being above the first threshold.

10. The circuitry (10) according to claim 9, wherein the stimulation artefact detection block (150) further comprises a delay circuit (190) for delaying a second control signal controlling the second switch (1402) in relation to a first control signal controlling the first switch (1401) and delaying a fourth control signal controlling the fourth switch (1404) in relation to a third control signal controlling the third switch (1403).

11. The circuitry according to claim 9 or 10, further comprising a third signal recording block (13) comprising a third signal recording amplifier (1103), a third feedback integrator (1303), a third multiplexer (1803), a third sampling circuit (1203), a fifth switch (1405) configured to selectively disconnect the output (116) of the third signal recording amplifier (1103) from an input node (126) of the third sampling circuit (1203), and a sixth switch (1406) connected to an output (127) of the third sampling circuit (1203), wherein the fifth and sixth switches (1405, 1406) are configured to be activated in dependence of the detection, by the stimulation artefact detection block (150), of the signal level of the stimulation artefact being below a second threshold, lower than the first threshold.

12. An apparatus (300) for recording a biological electrical signal, said apparatus (300) comprising one or more electrodes (304) for sensing the biological electrical signal and the circuitry (10) according to any one of the preceding claims.

13. A method for recording a biological electrical signal, said method comprising:
receiving (201), by a signal recording input, the biological electrical signal;
amplifying (202), by a signal recording amplifier connected to the signal recording input, the biological electrical signal;
sampling (203), by a sampling circuit having an input node connected to an output of the signal recording amplifier, the amplified biological electrical signal;
receiving (205), a control signal which is dependent of the detection of a stimulation artefact affecting recording of the biological electrical signal;
in response to receiving (205) the control signal, selectively disconnecting (206) the output of the signal recording amplifier from the input node of the sampling circuit using a switch; and
recording (207), the biological electrical signal, when the switch connects the output of the signal recording amplifier to the input node of the sampling circuit for output of the amplified biological signal with a subtracted DC offset; **characterised in that** the method further comprises : providing (204) a feedback signal into the signal recording amplifier, wherein the feedback signal is created by subtracting a direct current, DC, offset, from the amplified biological electrical signal, using a feedback integrator connected to the input node of the sampling circuit.

14. The method according to claim 13, further comprising generating the control signal, wherein said generating comprises:
receiving (208), by a stimulation artefact detection input, a reference biological signal corresponding to the biological signal to be recorded;
amplifying (209), by a stimulation artefact detection amplifier connected to the stimulation artefact detection input, the reference biological electrical signal;
comparing (210) the amplified reference biological electrical signal to a specified threshold;
generating (211) the control signal in dependence on the comparison of the amplified biological electrical signal and the specified threshold.

15. The method according to claim 13 or 14, further comprising
switching (212), using one or more multiplexers, between two or more different circuit configurations for single-ended or differential recording of the biological electrical signal.

## Patentansprüche

1. Schaltung (10) zum Aufzeichnen eines biologischen elektrischen Signals, wobei die Schaltung (10) umfasst:
einen Signalaufzeichnungseingang (100), der dazu eingerichtet ist, das biologische elektrische Signal zu empfangen;
einen Signalaufzeichnungsverstärker (110), der mit dem Signalaufzeichnungseingang (100) verbunden ist und dazu eingerichtet ist, ein verstärktes biologisches elektrisches Signal an einem Ausgangsknoten (111) des Signalaufzeichnungsverstärkers (110) bereitzustellen;
eine Abtastschaltung (120), die einen Eingangsknoten (121) aufweist, der mit dem Ausgangsknoten (111) des Verstärkers (110) verbunden ist und dazu eingerichtet ist, das verstärkte biologische elektrische Signal abzutasten;
einen Schalter (140), der dazu eingerichtet ist, den Ausgang (111) des Signalaufzeichnungsverstärkers (110) selektiv von dem Eingangsknoten (121) der Abtastschaltung (120) zu trennen; und
einen Stimulationsartefakt-Detektionsblock (150), der dazu eingerichtet ist, ein Stimulationsartefakt zu detektieren;
wobei der Schalter (140) dazu eingerichtet ist, ein Steuersignal zum Steuern des Schalters (140) zu empfangen, wobei das Steuersignal von einer Detektion, durch den Stimulationsartefakt-Detektionsblock (150), eines Stimulationsartefakts abhängt, das die Aufzeichnung des biologischen elektrischen Signals beeinträchtigt, **dadurch gekennzeichnet, dass** die Schaltung des Weiteren einen Rückkopplungsintegrator (130) umfasst, der mit dem Eingangsknoten (121) der Abtastschaltung (120) verbunden ist und dazu eingerichtet ist, ein Rückkopplungssignal in den Signalaufzeichnungsverstärker (110) einzuspeisen, wobei der Rückkopplungsintegrator (130) dazu eingerichtet ist, einen Gleichstromversatz von dem verstärkten biologischen elektrischen Signal zu subtrahieren.

2. Schaltung (10) nach Anspruch 1, wobei der Stimulationsartefakt-Detektionsblock (150) umfasst:
einen Stimulationsartefakt-Detektionseingang (152), der dazu eingerichtet ist, ein biologisches elektrisches Referenzsignal zu empfangen, das dem aufzuzeichnenden biologischen elektrischen Signal entspricht;
einen Stimulationsartefakt-Detektionsverstärker (154), der mit dem Stimulationsartefakt-Detektionseingang (152) verbunden ist und dazu eingerichtet ist, ein verstärktes biologisches elektrisches Referenzsignal an einem Ausgangsknoten (156) des Stimulationsartefakt-Detektionsverstärkers (154) bereitzustellen;
eine Komparatorschaltung (160), die einen Eingangsknoten (162) aufweist, der mit dem Ausgangsknoten (156) des Stimulationsartefakt-Detektionsverstärkers (154) verbunden ist und dazu eingerichtet ist, das verstärkte biologische elektrische Referenzsignal mit einer Schwelle zu vergleichen, wobei die Komparatorschaltung (160) dafür ausgelegt ist, das Stimulationsartefakt auf der Grundlage des Vergleichs des verstärkten biologischen elektrischen Referenzsignals und der spezifizierten Schwelle zu detektieren.

3. Schaltung (10) nach Anspruch 2, wobei die Komparatorschaltung (160) mit dem Schalter (140) verbunden ist; und
wobei der Schalter (140) dazu eingerichtet ist, das Steuersignal von der Komparatorschaltung (160) zu empfangen, wobei das Steuersignal auf der Detektion des Stimulationsartefakts durch die Komparatorschaltung (160) basiert.

4. Schaltung (10) nach einem der vorangehenden Ansprüche, des Weiteren umfassend einen Strombegrenzungs-Sicherheitsschaltungsblock (170) zwischen dem Signalaufzeichnungseingang (100) und dem Signalaufzeichnungsverstärker (110).

5. Schaltung (10) nach einem der vorangehenden Ansprüche, wobei die Abtastschaltung (120) ein Tiefpassfilter (122) umfasst, das dazu eingerichtet ist, das verstärkte biologische elektrische Signal an dem Eingangsknoten (121) abzutasten und zwischenzuspeichern.

6. Schaltung (10) nach einem der vorangehenden Ansprüche, wobei der Rückkopplungsintegrator (130) einen Operationsverstärker (132) und einen Rückkopplungskondensator (134), der zwischen einem Ausgang des Operationsverstärkers und einem invertierenden Eingang des Operationsverstärkers verbunden ist, um eine negative Rückkopplung zu dem Operationsverstärker (132) bereitzustellen, umfasst.

7. Schaltung (10) nach einem der vorangehenden Ansprüche, wobei der Signalaufzeichnungseingang (100) ein erster Signalaufzeichnungseingang (1001) ist, der dazu eingerichtet ist, ein erstes biologisches elektrisches Signal zu empfangen, und wobei die Schaltung (10) des Weiteren einen zweiten Signalaufzeichnungseingang (1002) umfasst, der dazu eingerichtet ist, ein zweites biologisches elektrisches Signal zu empfangen, wobei der Signalaufzeichnungsverstärker (110) ein Differenzverstärker ist, der einen invertierenden Eingang (112) und einen nicht-invertierenden Eingang (113) aufweist, die mit dem ersten Signalaufzeichnungseingang (1001) bzw. dem zweiten Signalaufzeichnungseingang (1002) verbunden sind, wobei der Rückkopplungsintegrator (130) mit einem Referenzeingangsport (114) des Signalaufzeichnungsverstärkers (110) verbunden ist.

8. Schaltung (10) nach einem der Ansprüche 1-6, wobei der Signalaufzeichnungseingang (100) ein erster Signalaufzeichnungseingang (1001) ist, der dazu eingerichtet ist, ein erstes biologisches elektrisches Signal zu empfangen, und wobei der erste Signalaufzeichnungseingang (1001) mit einem invertierenden Eingang (112) des Signalaufzeichnungsverstärkers (110) verbunden ist, wobei die Schaltung (10) des Weiteren einen zweiten Signalaufzeichnungseingang (1002) umfasst, der dazu eingerichtet ist, ein zweites biologisches elektrisches Signal zu empfangen, und wobei die Schaltung (10) des Weiteren einen Multiplexer (180) umfasst, wobei der Multiplexer (180) mit dem Rückkopplungsintegrator (130) verbunden ist und der Multiplexer (180) selektiv zwischen einer ersten und einer zweiten Konfiguration gesteuert wird; wobei in der ersten Konfiguration der Rückkopplungsintegrator (130) über den Multiplexer (180) mit einem nicht-invertierenden Eingang (113) des Signalaufzeichnungsverstärkers (110) verbunden ist und ein Referenzeingangsport (114) des Signalaufzeichnungsverstärkers (110) mit einer Gleichtaktspannung verbunden ist, und
wobei in der zweiten Konfiguration der Rückkopplungsintegrator (130) über den Multiplexer (180) mit dem Referenzeingangsport (114) des Signalaufzeichnungsverstärkers (110) verbunden ist und der zweite Signalaufzeichnungseingang (1002) mit dem nicht-invertierenden Eingang (113) des Signalaufzeichnungsverstärkers (110) verbunden ist.

9. Schaltung (10) nach Anspruch 8, wobei der Signalaufzeichnungsverstärker (110) ein erster Signalaufzeichnungsverstärker (1101) ist, der Rückkopplungsintegrator (130) ein erster Rückkopplungsintegrator (1301) ist, der Schalter (140) ein erster Schalter (1401) ist, der Multiplexer (180) ein erster Multiplexer (1801) ist, und die Abtastschaltung (120) eine erste Abtastschaltung (1201) ist, wobei der erste Signalaufzeichnungsverstärker (1101), der erste Rückkopplungsintegrator (1301), der erste Schalter (1401), der erste Multiplexer (1801), die erste Abtastschaltung (1201) und ein zweiter Schalter (1402), der mit einem Ausgang (123) der ersten Abtastschaltung (1201) verbunden ist, einen ersten Signalaufzeichnungsblock (11) bilden, wobei die Schaltung (10) des Weiteren einen zweiten Signalaufzeichnungsblock (12) umfasst, der einen zweiten Signalaufzeichnungsverstärker (1102), einen zweiten Rückkopplungsintegrator (1302), einen zweiten Multiplexer (1802), eine zweite Abtastschaltung (1202), einen dritten Schalter (1403), der dazu eingerichtet ist, den Ausgang (115) des zweiten Signalaufzeichnungsverstärkers (1102) selektiv von einem Eingangsknoten (124) der zweiten Abtastschaltung (1202) zu trennen, und einen vierten Schalter (1404), der mit einem Ausgang (125) der zweiten Abtastschaltung (1202) verbunden ist, umfasst, wobei der erste und der zweite Schalter (1401, 1402) dazu eingerichtet sind, in Abhängigkeit davon deaktiviert zu werden, dass die Detektion, durch den Stimulationsartefakt-Detektionsblock (150), eines Signalpegels des Stimulationsartefakts über einer ersten Schwelle liegt, und der dritte und der vierte Schalter (1403, 1404) dazu eingerichtet sind, in Abhängigkeit davon aktiviert zu werden, dass die Detektion, durch den Stimulationsartefakt-Detektionsblock (150), des Signalpegels des Stimulationsartefakts über der ersten Schwelle liegt.

10. Schaltung (10) nach Anspruch 9, wobei der Stimulationsartefakt-Detektionsblock (150) des Weiteren eine Verzögerungsschaltung (190) umfasst, um ein zweites Steuersignal, das den zweiten Schalter (1402) steuert, in Bezug auf ein erstes Steuersignal, das den ersten Schalter (1401) steuert, zu verzögern und ein viertes Steuersignal, das den vierten Schalter (1404) steuert, in Bezug auf ein drittes Steuersignal, das den dritten Schalter (1403) steuert, zu verzögern.

11. Schaltung nach Anspruch 9 oder 10, des Weiteren umfassend einen dritten Signalaufzeichnungsblock (13), der einen dritten Signalaufzeichnungsverstärker (1103), einen dritten Rückkopplungsintegrator (1303), einen dritten Multiplexer (1803), eine dritte Abtastschaltung (1203), einen fünften Schalter (1405), der dazu eingerichtet ist, den Ausgang (116) des dritten Signalaufzeichnungsverstärkers (1103) selektiv von einem Eingangsknoten (126) der dritten Abtastschaltung (1203) zu trennen, und einen sechsten Schalter (1406), der mit einem Ausgang (127) der dritten Abtastschaltung (1203) verbunden ist, umfasst, wobei der fünfte und der sechste Schalter (1405, 1406) dazu eingerichtet sind, in Abhängigkeit davon aktiviert zu werden, dass die Detektion, durch den Stimulationsartefakt-Detektionsblock (150), des Signalpegels des Stimulationsartefakts unter einer zweiten Schwelle liegt, die niedriger als die erste Schwelle ist.

12. Vorrichtung (300) zum Aufzeichnen eines biologischen elektrischen Signals, wobei die Vorrichtung (300) eine oder mehrere Elektroden (304) zum Abfühlen des biologischen elektrischen Signals und die Schaltung (10) nach einem der vorangehenden Ansprüche umfasst.

13. Verfahren zum Aufzeichnen eines biologischen elektrischen Signals, wobei das Verfahren umfasst:
Empfangen (201), durch einen Signalaufzeichnungseingang, des biologischen elektrischen Signals;
Verstärken (202), durch einen mit dem Signalaufzeichnungseingang verbundenen Signalaufzeichnungsverstärker, des biologischen elektrischen Signals;
Abtasten (203), durch eine Abtastschaltung, die einen Eingangsknoten aufweist, der mit einem Ausgang des Signalaufzeichnungsverstärkers verbunden ist, des verstärkten biologischen elektrischen Signals;
Empfangen (205) eines Steuersignals, das von der Detektion eines Stimulationsartefakts abhängt, das die Aufzeichnung des biologischen elektrischen Signals beeinträchtigt;
in Reaktion auf das Empfangen (205) des Steuersignals, selektives Trennen (206) des Ausgangs des Signalaufzeichnungsverstärkers von dem Eingangsknoten der Abtastschaltung unter Verwendung eines Schalters; und
Aufzeichnen (207) des biologischen elektrischen Signals, wenn der Schalter den Ausgang des Signalaufzeichnungsverstärkers mit dem Eingangsknoten der Abtastschaltung verbindet, um das verstärkte biologische Signal mit einem subtrahierten Gleichstromversatz auszugeben; **dadurch gekennzeichnet, dass** das Verfahren des Weiteren umfasst: Einspeisen (204) eines Rückkopplungssignals in den Signalaufzeichnungsverstärker, wobei das Rückkopplungssignal durch Subtrahieren eines Gleichstromversatzes von dem verstärkten biologischen elektrischen Signal unter Verwendung eines mit dem Eingangsknoten der Abtastschaltung verbundenen Rückkopplungsintegrators erzeugt wird.

14. Verfahren nach Anspruch 13, des Weiteren umfassend das Generieren des Steuersignals, wobei das Generieren umfasst:
Empfangen (208), durch einen Stimulationsartefakt-Detektionseingang, eines biologischen Referenzsignals, das dem aufzuzeichnenden biologischen Signal entspricht;
Verstärken (209), durch einen mit dem Stimulationsartefakt-Detektionseingang verbundenen Stimulationsartefakt-Detektionsverstärker, des biologischen elektrischen Referenzsignals;
Vergleichen (210) des verstärkten biologischen elektrischen Referenzsignals mit einer spezifizierten Schwelle;
Generieren (211) des Steuersignals in Abhängigkeit von dem Vergleich des verstärkten biologischen elektrischen Signals und der spezifizierten Schwelle.

15. Verfahren nach Anspruch 13 oder 14, des Weiteren umfassend: Umschalten (212), unter Verwendung eines oder mehrerer Multiplexer, zwischen zwei oder mehr verschiedenen Schaltungskonfigurationen für eine ein-endige oder differentielle Aufzeichnung des biologischen elektrischen Signals.

## Revendications

1. Circuit (10) pour l'enregistrement d'un signal électrique biologique, ledit circuit (10) comprenant :
une entrée d'enregistrement de signal (100) configurée pour recevoir le signal électrique biologique ;
un amplificateur d'enregistrement de signal (110) connecté à l'entrée d'enregistrement de signal (100) et configuré pour fournir un signal électrique biologique amplifié à un nœud de sortie (111) de l'amplificateur d'enregistrement de signal (110) ;
un circuit d'échantillonnage (120) ayant un nœud d'entrée (121) connecté au nœud de sortie (111) de l'amplificateur (110) et configuré pour échantillonner le signal électrique biologique amplifié ;
un commutateur (140) configuré pour déconnecter sélectivement la sortie (111) de l'amplificateur d'enregistrement de signal (110) du nœud d'entrée (121) du circuit d'échantillonnage (120) ; et
un bloc de détection d'artefacts de stimulation (150) configuré pour détecter un artefact de stimulation ;
dans lequel le commutateur (140) est configuré pour recevoir un signal de commande pour commander le commutateur (140), dans lequel le signal de commande dépend de la détection, par le bloc de détection d'artefacts de stimulation (150), d'un artefact de stimulation affectant l'enregistrement de signal électrique biologique, **caractérisé en ce que** le circuit comprend en outre un intégrateur à rétroaction (130) connecté au noeud d'entrée (121) du circuit d'échantillonnage (120) et configuré pour fournir un signal de rétroaction à l'amplificateur d'enregistrement de signal (110), dans lequel l'intégrateur à rétroaction (130) est configuré pour soustraire un courant continu, DC, décalé de signal électrique biologique amplifié.

2. Circuit (10) selon la revendication 1, dans lequel le bloc de détection d'artefacts de stimulation (150) comprend :
une entrée de détection d'artefacts de stimulation (152) configurée pour recevoir un signal électrique biologique de référence correspondant au signal électrique biologique à enregistrer ;
un amplificateur de détection d'artefacts de stimulation (154) connecté à l'entrée de détection d'artefacts de stimulation (152) et configuré pour fournir un signal électrique biologique de référence amplifié à un nœud de sortie (156) de l'amplificateur de détection d'artefacts de stimulation (154) ;
un circuit comparateur (160) comportant un nœud d'entrée (162) connecté au nœud de sortie (156) de l'amplificateur de détection d'artefacts de stimulation (154) et configuré pour comparer le signal électrique biologique de référence amplifié à un seuil, dans lequel le circuit comparateur (160) est agencé pour détecter l'artefact de stimulation sur la base de la comparaison de signal électrique biologique de référence amplifié avec le seuil spécifié.

3. Circuit (10) selon la revendication 2, dans lequel le circuit comparateur (160) est connecté au commutateur (140) ; et
dans lequel le commutateur (140) est configuré pour recevoir le signal de commande provenant du circuit comparateur (160), dans lequel le signal de commande est basé sur la détection de l'artefact de stimulation par le circuit comparateur (160).

4. Circuit (10) selon une quelconque des revendications précédentes, comprenant en outre un bloc de circuit de sécurité de limitation de courant (170) placé entre l'entrée d'enregistrement de signal (100) et l'amplificateur d'enregistrement de signal (110).

5. Circuit (10) selon une quelconque des revendications précédentes, dans lequel le circuit d'échantillonnage (120) comprend un filtre passe-bas (122) configuré pour échantillonner et maintenir le signal électrique biologique amplifié au niveau du nœud d'entrée (121).

6. Circuit (10) selon une quelconque des revendications précédentes, dans lequel l'intégrateur à rétroaction (130) comprend un amplificateur opérationnel (132) et un condensateur de rétroaction (134) connecté entre une sortie de l'amplificateur opérationnel et une entrée inverseuse de l'amplificateur opérationnel afin de fournir une rétroaction négative à l'amplificateur opérationnel (132).

7. Circuit (10) selon une quelconque des revendications précédentes, dans lequel l'entrée d'enregistrement de signal (100) est une première entrée d'enregistrement de signal (1001) configurée pour recevoir un premier signal électrique biologique et dans lequel le circuit (10) comprend en outre une deuxième entrée d'enregistrement de signal (1002) configurée pour recevoir un deuxième signal électrique biologique, dans lequel l'amplificateur d'enregistrement de signal (110) est un amplificateur différentiel possédant une entrée inverseuse (112) et une entrée non inverseuse (113) connectées respectivement à la première entrée d'enregistrement de signal (1001) et à la deuxième entrée d'enregistrement de signal (1002), dans lequel l'intégrateur à rétroaction (130) est connecté à un port d'entrée de référence (114) de l'amplificateur d'enregistrement de signal (110).

8. Circuit (10) selon une quelconque des revendications 1 à 6, dans lequel l'entrée d'enregistrement de signal (100) est une première entrée d'enregistrement de signal (1001) configurée pour recevoir un premier signal électrique biologique et dans lequel la première entrée d'enregistrement de signal (1001) est connectée à une entrée inverseuse (112) de l'amplificateur d'enregistrement de signal (110), dans lequel le circuit (10) comprend en outre une deuxième entrée d'enregistrement de signal (1002) configurée pour recevoir un deuxième signal électrique biologique, et dans lequel le circuit (10) comprend en outre un multiplexeur (180), dans lequel le multiplexeur (180) est connecté à l'intégrateur à rétroaction (130) et le multiplexeur (180) est commandé sélectivement entre une première et une deuxième configuration;
dans lequel, dans la première configuration, l'intégrateur à rétroaction (130) est connecté, via le multiplexeur (180), à une entrée non inverseuse (113) de l'amplificateur d'enregistrement de signal (110) et un port d'entrée de référence (114) de l'amplificateur d'enregistrement de signal (110) est connecté à une tension de mode commun et
dans lequel, dans la deuxième configuration, l'intégrateur à rétroaction (130) est connecté, via le multiplexeur (180), au port d'entrée de référence (114) de l'amplificateur d'enregistrement de signal (110) et la deuxième entrée d'enregistrement de signal (1002) est connectée à l'entrée non inverseuse (113) de l'amplificateur d'enregistrement de signal (110).

9. Circuit (10) selon la revendication 8, dans lequel l'amplificateur d'enregistrement de signal (110) est un premier amplificateur d'enregistrement de signal (1101), l'intégrateur à rétroaction (130) est un premier intégrateur à rétroaction (1301), le commutateur (140) est un premier commutateur (1401), le multiplexeur (180) est un premier multiplexeur (1801) et le circuit d'échantillonnage (120) est un premier circuit d'échantillonnage (1201), dans lequel le premier amplificateur d'enregistrement de signal (1101), le premier intégrateur à rétroaction (1301), le premier commutateur (1401), le premier multiplexeur (1801), le premier circuit d'échantillonnage (1201) et un deuxième commutateur (1402) connecté à une sortie (123) du premier circuit d'échantillonnage (1201) forment un premier bloc d'enregistrement de signal (11), dans lequel le circuit (10) comprend en outre un deuxième bloc d'enregistrement de signal (12) comprenant un un deuxième amplificateur d'enregistrement de signal (1102), un deuxième intégrateur à rétroaction (1302), un deuxième multiplexeur (1802), un deuxième circuit d'échantillonnage (1202), un troisième commutateur (1403) configuré pour déconnecter sélectivement la sortie (115) du deuxième amplificateur d'enregistrement de signal (1102) d'un nœud d'entrée (124) du deuxième circuit d'échantillonnage (1202) et un quatrième commutateur (1404) connecté à une sortie (125) du deuxième circuit d'échantillonnage (1202), dans lequel les premier et deuxième commutateurs (1401, 1402) sont configurés pour être désactivés en fonction de la détection, par le bloc de détection d'artefacts de stimulation (150), d'un niveau de signal de l'artefact de stimulation supérieur à un premier seuil et les troisième et quatrième commutateurs (1403, 1404) sont configurés pour être activés en fonction de la détection, par le bloc de détection d'artefacts de stimulation (150), du niveau de signal de l'artefact de stimulation dépassant le premier seuil.

10. Circuit (10) selon la revendication 9, dans lequel le bloc de détection d'artefact de stimulation (150) comprend en outre un circuit de temporisation (190) pour retarder un deuxième signal de commande commandant le deuxième interrupteur (1402) par rapport à un premier signal de commande commandant le premier interrupteur (1401) et retarder un quatrième signal de commande commandant le quatrième interrupteur (1404) par rapport à un troisième signal de commande commandant le troisième interrupteur (1403).

11. Circuit selon la revendication 9 ou 10, comprenant en outre un troisième sbloc d'enregistrement de signal (13) comprenant un troisième amplificateur d'enregistrement de signal (1103), un troisième intégrateur à rétroaction (1303), un troisième multiplexeur (1803), un troisième circuit d'échantillonnage (1203), un cinquième commutateur (1405) configuré pour déconnecter sélectivement la sortie (116) du troisième amplificateur d'enregistrement de signal (1103) d'un nœud d'entrée (126) du troisième circuit d'échantillonnage (1203), et un sixième commutateur (1406) connecté à une sortie (127) du troisième circuit d'échantillonnage (1203), dans lequel les cinquième et sixième commutateurs (1405, 1406) sont configurés pour être activés en fonction de la détection, par le bloc de détection d'artefacts de stimulation (150), du niveau de signal de l'artefact de stimulation étant inférieur à un deuxième seuil, inférieur au premier seuil.

12. Appareil (300) d'enregistrement d'un signal électrique biologique, ledit appareil (300) comprenant une ou plusieurs électrodes (304) pour la détection de signal électrique biologique et le circuit (10) selon une quelconque des revendications précédentes.

13. Procédé d'enregistrement d'un signal électrique biologique, ledit procédé comprenant :
la réception (201), par une entrée d'enregistrement de signal, du signal électrique biologique ;
l'amplification (202), par un amplificateur d'enregistrement de signal connecté à l'entrée d'enregistrement de signal, de signal électrique biologique ;
l'échantillonnage (203), par un circuit d'échantillonnage dont un nœud d'entrée est connecté à une sortie de l'amplificateur d'enregistrement de signal, du signal électrique biologique amplifié ;
la réception (205), d'un signal de commande dépendant de la détection d'un artefact de stimulation affectant l'enregistrement de signal électrique biologique;
en réponse à la réception (205) du signal de commande, la déconnexion sélective (206) de la sortie de l'amplificateur d'enregistrement de signal du nœud d'entrée du circuit d'échantillonnage à l'aide d'un commutateur; et
l'enregistrement (207) de signal électrique biologique, lorsque le commutateur connecte la sortie de l'amplificateur d'enregistrement de signal au nœud d'entrée du circuit d'échantillonnage pour la sortie du signal biologique amplifié avec un décalage de courant continu, DC, soustrait;
**caractérisé en ce que** le procédé comprend en outre : la fourniture (204) d'un signal de rétroaction dans l'amplificateur d'enregistrement de signal, dans lequel le signal de rétroaction est créé en soustrayant un décalage de courant continu, DC, du signal électrique biologique amplifié, à l'aide d'un intégrateur à rétroaction connecté au nœud d'entrée du circuit d'échantillonnage.

14. Procédé selon la revendication 13, comprenant en outre la génération du signal de commande, dans lequel ladite génération comprend:
la réception (208), par une entrée de détection d'artefacts de stimulation, d'un signal biologique de référence correspondant au signal biologique à enregistrer ;
l'amplification (209), par un amplificateur de détection d'artefacts de stimulation connecté à l'entrée de détection d'artefacts de stimulation, du signal électrique biologique de référence ;
la comparaison (210) du signal électrique biologique de référence amplifié avec un seuil spécifié ;
la génération (211) du signal de commande en fonction de la comparaison du signal électrique biologique amplifié et du seuil spécifié.

15. Procédé selon la revendication 13 ou 14, comprenant en outre :
la commutation (212), à l'aide d'un ou plusieurs multiplexeurs, entre deux ou plusieurs configurations de circuit différentes pour l'enregistrement asymétrique ou différentiel du signal électrique biologique.
